# FASCICULE DE BREVET EUROPEEN

(11) **EP 4 319 647 B1**
(45) Date de publication et mention de la délivrance du brevet: **14.01.2026**
(21) Numéro de dépôt: 22718742.4
(22) Date de dépôt: 31.03.2022
(51) Int. Cl.: A61B 17/04

(54) **ANCRE DE SUTURE POUR UN ANCRAGE D'UN TISSU MOU SUR UN OS**
NAHTANKER ZUR VERANKERUNG VON WEICHGEWEBE AN EINEM KNOCHEN
SUTURE ANCHOR FOR ANCHORING SOFT TISSUE TO A BONE

(30) Priorité: 07.04.2021 FR 2103551
(43) Date de publication de la demande: 14.02.2024
(73) Titulaire: RS4, 26000 Valence (FR)
(72) Inventeur: BATAILLE, Jean-François, 30650 ROCHEFORT DU GARD (FR); BELLUMORE, Yves, 31600 MURET (FR); BOUDARD, Guillaume, 25170 RECOLOGNE (FR); GADEA, François, 83000 TOULON (FR); GEORGE, Thierry, 45160 OLIVET (FR); GUIGNAND, Didier, 69370 SAINT DIDIER AU MONT D'OR (FR); LE RUE, Olivier, 59800 LILLE (FR); MOREEL, Philippe, 13710 FUVEAU (FR); NAVEZ, Grégory, 26000 VALENCE (FR); SARRAN, Olivier, 64140 BILLERE (FR); TOURNIER, Clément, 33000 BORDEAUX (FR)
(74) Mandataire: Germain Maureau
(86) Numéro de dépôt international: PCT/FR2022/050609
(87) Numéro de publication internationale: WO 2022/214756

(56) Documents cités:
- IT-A1- 201800 020 884
- US-A1- 2016 144 066
- US-A1- 2020 078 004

## Description

L'invention se rapporte à une ancre de suture pour un ancrage d'un tissu mou sur un os, et en particulier à une ancre de suture comprenant un fil de suture accouplé à un élément d'ancrage compressible.

L'invention vise plus particulièrement à fournir une ancre de suture permettant la fixation d'un fil de suture sur un os, un tel fil de suture étant prévu pour être relié à un tissu mou pour amarrer le tissu mou sur sa zone d'amarrage osseuse native (aussi appelée zone d'insertion osseuse) ; étant entendu par « tissu mou » toute partie d'un corps humain ou animal formant un tissu de soutien extra-squelettique servant à supporter une structure ou un organe, et en particulier un ligament, un tendon et un muscle.

Une application favorite, et non limitative, concerne l'ancrage des tendons de la coiffe des rotateurs de l'épaule sur leur zone d'amarrage osseuse native autour de la tête humérale, étant noté que d'autres applications sont envisageables dans l'ancrage de tendon ou de ligament au niveau d'autres articulations telles que la hanche, le genou, le coude, la cheville ou le poignet.

Dans le domaine de la coiffe des rotateurs de l'épaule, il est classique d'opérer sous arthroscopie selon une technique dite de double rang consistant à :
- ancrer une première rangée d'ancres de suture dites de premier rang qui assurent un ancrage et un blocage solide de fils de suture qui serviront à suturer et à amarrer les tendons sur leur zone d'amarrage osseuse native, puis
- ancrer une seconde rangée d'ancres de suture dites de second rang, en position latérale par rapport à la première rangée, qui servent à fixer sur l'os les parties libres des brins des fils de suture et ainsi les tendre pour plaquer les tendons contre la zone d'amarrage osseuse native et donc favoriser la cicatrisation.

L'invention est applicable pour former une ancre de premier rang ou une ancre de second rang, selon la méthode chirurgicale employée.

Dans le domaine des ancres de suture, il est connu d'employer un élément d'ancrage compressible accouplé à un fil de suture, où l'élément d'ancrage compressible a une structure en accordéon déformable pour pouvoir être comprimé une fois inséré dans un trou ménagé dans l'os et ainsi être coincé dans le trou.

L'état de la technique peut être illustré par les enseignements des documents EP2572650A1 et WO2012/103536 qui décrivent chacun une ancre de suture comprenant, d'une part, un élément d'ancrage compressible se présentant au repos sous la forme d'un corps longiligne conformée en accordéon ou en serpentin en ayant des sections courbées successives et, d'autre part, un fil de suture présentant deux brins qui traversent de manière quasi rectiligne et parallèle les sections courbées de l'élément d'ancrage compressible. Il est également connu du document EP3277331 d'employer une ancre de suture comprenant, d'une part, un élément d'ancrage compressible se présentant au repos sous la forme d'un corps tubulaire en « U » et, d'autre part, un fil de suture qui pénètre dans le corps tubulaire via une première ouverture et ressort via une autre ouverture.

Dans ces différentes solutions connues, une fois l'élément d'ancrage compressible inséré dans le trou, il suffit de tirer sur les deux brins du fil de suture dépassant du trou pour que l'élément d'ancrage compressible se comprime et forme un amas qui se bloque dans le trou.

Il est également connu des documents US2016/144066 et IT 2018 0002 0884 d'employer une gaine compressible en forme de « U », à l'intérieur de laquelle peut coulisser un fil de suture, et où des fenêtres sont ménagées l'une en face de l'autre dans les sections longitudinales de la gaine pour former des plis saillants une fois la gaine comprimée.

Cependant, de telles solutions ne sont pas totalement satisfaisantes en terme de résistance à l'arrachement, les conformations au repos de l'élément d'ancrage compressible et du fil de suture conduisant, par traction sur le fil de suture, à une compression de l'élément d'ancrage compressible telle que les efforts appliqués par ce dernier sur les parois internes du trou sont mal maîtrisés et ainsi généralement mal répartis.

La présente invention vise à résoudre en tout ou partie les problèmes précités, en proposant une ancre de suture dont l'élément d'ancrage compressible et le fil de suture sont conformés au repos pour permettre, par traction sur le fil de suture, de comprimer l'élément d'ancrage compressible avec une uniformisation améliorée des appuis de l'élément d'ancrage compressible sur les parois internes du trou, et donc une meilleure répartition des contraintes, procurant ainsi une meilleure résistance à l'arrachement par une meilleure répartition de l'appui.

A cette fin, l'invention propose une ancre de suture, pour un ancrage d'un tissu mou sur un os, une telle ancre de suture comprenant au moins un fil de suture accouplé à un élément d'ancrage compressible, ledit élément d'ancrage compressible comprenant au moins une gaine compressible traversée par ce fil de suture, cette ancre de suture étant configurable entre :
- une configuration de repos dans laquelle l'élément d'ancrage compressible est au repos ; et
- une configuration comprimée dans laquelle l'élément d'ancrage compressible est comprimé,

dans laquelle, en configuration de repos :
   - la gaine compressible présente une forme générale d'arche en « U » avec une section coudée centrale reliant entre elles une première section longitudinale et une seconde section longitudinale espacées l'une de l'autre et se terminant par des terminaisons libres munies d'une première ouverture et d'une seconde ouverture respectives ; et
   - le fil de suture est libre de coulisser à l'intérieur de la gaine compressible et présente un premier brin entrant à l'intérieur de la première section longitudinale de la gaine compressible au travers de la première ouverture, et un second brin prolongeant le premier brin et ressortant de la seconde section longitudinale de la gaine compressible au travers de la seconde ouverture ;
et dans laquelle la gaine compressible présente :
   - une première fenêtre disposée dans la première section longitudinale et ouvrant sur le premier brin ; et
   - une seconde fenêtre disposée dans la seconde section longitudinale et ouvrant sur le second brin,
où, en configuration de repos, la première fenêtre et la seconde fenêtre de la gaine compressible sont disposées de part et d'autre d'un plan médian de la gaine compressible dans lequel sont inclus la section coudée centrale, la première section longitudinale et la seconde section longitudinale de ladite gaine compressible.

Ainsi, l'invention propose que le fil de suture (ou chaque fil de suture) traverse la gaine compressible sur toute sa longueur, permettant à cette gaine compressible (et donc à l'élément d'ancrage compressible) de pouvoir passer de la configuration de repos à la configuration comprimée sous l'effet d'une tension exercée par le fil de suture en tirant sur les deux brins. L'avantage des deux fenêtres est qu'elles forment des amorces de pliage pour les deux sections longitudinales de la gaine compressible, autrement dit des zones de déformation (ou de compression) privilégiées. Ainsi, une fois comprimée, la gaine compressible présente deux plis saillants au niveau des fenêtres, du fait de la compression de ses deux sections longitudinales. Ces deux plis saillants sont disposés de part et d'autre du plan médian, offrant ainsi une répartition équilibrée des efforts (ou appuis) sur les parois internes du trou osseux.

Par ailleurs, ces deux plis saillants vont former des points d'appui diamétralement opposés, qui vont contribuer à une mise en rotation des deux sections longitudinales de la gaine compressible, concomitamment à la compression axiale de ces deux sections longitudinales. Cette combinaison d'une rotation et d'une compression axiale conduit à ce que les deux sections longitudinales s'enroulent l'une autour de l'autre, de sorte que ces deux sections longitudinales sont à la fin comprimées l'une contre l'autre selon une conformation en hélice. Une telle conformation en hélice offre une répartition relativement uniforme des efforts (ou appuis) sur les parois internes du trou. L'invention permet donc une compression maitrisée et équilibrée en longueur et en largeur.

En configuration de repos, l'élément d'ancrage compressible (ou la gaine compressible) présente une première valeur de dimension longitudinale et une première valeur de dimension transversale tandis qu'en configuration comprimée, l'élément d'ancrage compressible présente une seconde valeur de dimension longitudinale inférieure à la première valeur de dimension longitudinale, et une seconde valeur de dimension transversale supérieure à la première valeur de dimension transversale. Autrement dit, en configuration comprimée l'élément d'ancrage compressible est plus court (dimension longitudinale mesurée selon une direction longitudinale correspondant à la direction du trou qui est rectiligne et donc aussi à la direction d'insertion de l'ancre dans le trou) et plus large (dimension transversale orthogonale à la direction longitudinale).

Selon une caractéristique, le premier brin et le second brin du fil de suture sont également libres de coulisser dans les deux sections longitudinales de la gaine compressible dans la configuration comprimée.

Autrement dit, lorsque l'élément d'ancrage compressible est comprimé (et donc coincé) dans le trou osseux, le fil de suture peut toujours coulisser, que ce soit en tirant sur le premier brin ou sur le second brin.

Selon une caractéristique, la gaine compressible présente une fenêtre centrale disposée dans la section coudée centrale, sur une portion intérieure de coude, ouvrant sur une section coudée du fil de suture reliant le premier brin au second brin.

Ainsi, lorsqu'on tire sur les deux brins du fil de suture, la section coudée du fil de suture remonte et vient plus vite comprimer les deux sections longitudinales pour former les plus saillants au niveau des fenêtres.

Selon une autre caractéristique, en configuration de repos, la première fenêtre et la seconde fenêtre s'étendent chacune sur une longueur de fenêtre mesurée dans le sens de la longueur de la première section longitudinale et de la seconde section longitudinale respectives, où cette longueur de fenêtre est de même valeur pour la première fenêtre et la seconde fenêtre, à plus ou moins 10% près.

Avantageusement, en configuration de repos, la gaine compressible présente une première valeur de dimension longitudinale mesurée longitudinalement depuis la section coudée centrale jusqu'à la terminaison libre de la première section longitudinale ou de la seconde section longitudinale, et le ratio entre la longueur de fenêtre et la première valeur de dimension longitudinale est compris entre 0,1 et 0,4.

Selon une possibilité, la gaine compressible reçoit intérieurement un ou deux fils de suture.

Dans une réalisation particulière, l'élément d'ancrage compressible comprend une seule gaine compressible.

Selon une possibilité, l'élément d'ancrage compressible comprend un anneau extensible solidaire de la gaine compressible, ledit anneau extensible étant disposé autour de la gaine compressible et venant en regard de la première fenêtre et de la seconde fenêtre.

Un tel anneau extensible présente l'avantage que, lorsque la gaine compressible est comprimée et que les plis saillants se forment, ces plis saillants vont s'élargir et vont pousser sur l'anneau. Ainsi, cet anneau va se déformer transversalement, sous la contrainte des plis saillants, contribuant à une répartition des appuis sur toute la périphérie de l'anneau.

Dans une autre réalisation particulière, l'élément d'ancrage compressible comprend deux gaines compressibles solidaires l'une de l'autre au niveau de leurs sections coudées centrales respectives et dont les plans médians sont orthogonaux, chaque gaine compressible recevant intérieurement au moins un fil de suture.

Ainsi, avec deux gaines, l'élément d'ancrage compressible présentera quatre plis saillants une fois comprimé, favorisant une répartition des appuis sur la périphérie de l'élément d'ancrage compressible.

Selon une caractéristique, la gaine compressible de l'élément d'ancrage compressible comprend un corps tubulaire qui est tissé ou tressé avec des fibres réalisées dans un ou plusieurs matériaux biocompatibles.

Selon une autre caractéristique, le fil de suture comprend un corps qui est tissé ou tressé avec des fibres réalisées dans un ou plusieurs matériaux biocompatibles.

Dans un mode de réalisation particulier, les fibres du corps de la gaine compressible et les fibres du corps du fil de suture sont réalisées dans au moins un même matériau biocompatible.

Selon une caractéristique, les fibres - que ce soit celles du corps de la gaine compressible et/ou celles du corps du fil de suture - sont réalisées dans un ou plusieurs matériaux biocompatibles choisis dans la liste comprenant : polyéthylène, polyester, polyhydroxyalkanoate, et une combinaison d'au moins certains d'entre eux.

Selon une autre caractéristique, les fibres sont réalisées au moins dans le matériau biocompatible qui est du polyéthylène à très haut poids moléculaire dit « UHMWPE ».

Selon une variante, les fibres - que soit celles du corps de la gaine compressible et/ou celles du corps du fil de suture - sont réalisées dans un ou plusieurs matériaux biocompatibles et résorbables.

Dans une réalisation avantageuse, les sections longitudinales de la gaine compressible présentent un premier diamètre et le fil de suture présente un second diamètre qui est inférieur au premier diamètre, avec un quotient du premier diamètre sur le second diamètre qui est au moins supérieur à 2.

Selon une variante, la gaine compressible est un corps longiligne ayant un premier diamètre constant sur toute sa longueur et qui est recourbé pour présenter au repos la forme générale d'arche en « U » et qui est maintenu recourbé au moyen du fil de suture qui le traverse selon le déploiement en double hélice.

Avantageusement, la première section longitudinale et la seconde section longitudinale de la gaine compressible sont comprimées en présentant un premier pli saillant et un second pli saillant formés respectivement au niveau de la première fenêtre et de la seconde fenêtre.

Dans un mode de réalisation particulier, en configuration comprimée, la première section longitudinale et la seconde section longitudinale de la gaine compressible sont comprimées l'une contre l'autre selon une conformation en hélice.

L'invention se rapporte également à un kit de suture, pour un ancrage d'un tissu mou sur un os, un tel kit de suture comprenant au moins une ancre de suture selon l'invention, et au moins un instrument d'insertion propre à insérer cette ancre de suture lorsqu'en configuration de repos à l'intérieur d'un trou ménagé dans l'os.

Selon une possibilité, l'instrument d'insertion comprend une partie distale propre à passer entre la première section longitudinale et la seconde section longitudinale de la gaine compressible, et se terminant par une extrémité libre propre à venir en contact avec la section coudée centrale de la gaine compressible afin de prendre appui sur celle-ci et permettre d'exercer un effort de poussée.

Selon une autre possibilité, l'extrémité libre de l'instrument d'insertion se présente sous la forme d'une fourche à deux bras.

Avantageusement, les deux bras se terminent par des pointes qui dépassent de la section coudée centrale de la gaine compressible.

Ainsi l'extrémité libre de l'instrument d'insertion forme une extrémité auto-perforante, permettant de ménager le trou par impaction en même temps que l'ancre de suture est inséré dans le trou, ce qui est avantageux pour ne pas avoir à localiser le trou pour insérer l'ancre de suture.

Selon une autre possibilité, le kit de suture comprend également un tube de guidage propre à venir en appui sur les terminaisons libres de la première section longitudinale et de la seconde section longitudinale de la gaine compressible, et présentant un canal intérieur longitudinal dimensionné pour un passage du premier brin et du second brin du fil de suture et de l'instrument d'insertion.

D'autres caractéristiques et avantages de la présente invention apparaîtront à la lecture de la description détaillée ci-après, de trois exemples de mise en œuvre non limitatif, faite en référence aux figures annexées dans lesquelles :
[Fig 1] est une vue schématique d'une première ancre de suture selon l'invention en configuration de repos, avec une illustration en perspective à gauche et de côté à droite ;
[Fig 2] est une vue schématique de dessus de la première ancre de suture de la Figure 1 en configuration de repos ;
[Fig 3] est une vue schématique de la première ancre de suture de la Figure 1 en configuration comprimée, avec une illustration en perspective à gauche et une illustration de côté à droite ;
[Fig 4] est une vue schématique de dessus de la première ancre de suture de la Figure 1 en configuration comprimée ;
[Fig 5] est une vue schématique d'une deuxième ancre de suture selon l'invention en configuration de repos, avec une illustration en perspective à gauche et de côté à droite ;
[Fig 6] est une vue schématique de dessus de la deuxième ancre de suture de la Figure 5 en configuration de repos ;
[Fig 7] est une vue schématique de la deuxième ancre de suture de la Figure 5 en configuration comprimée, avec une illustration en perspective à gauche et une illustration de côté à droite ;
[Fig 8] est une vue schématique de dessus de la deuxième ancre de suture de la Figure 5 en configuration comprimée ;
[Fig 9] est une vue schématique de dessus d'une troisième ancre de suture en configuration de repos ;
[Fig 10] est une vue schématique de la troisième ancre de suture de la Figure 9 en configuration de repos, avec une illustration en perspective à gauche et de côté à droite ;
[Fig 11] est une vue schématique de la troisième ancre de suture de la Figure 9 en configuration comprimée, avec une illustration en perspective à gauche et une illustration de côté à droite ;
[Fig 12] est une vue schématique de dessus de la troisième ancre de suture de la Figure 9 en configuration comprimée.

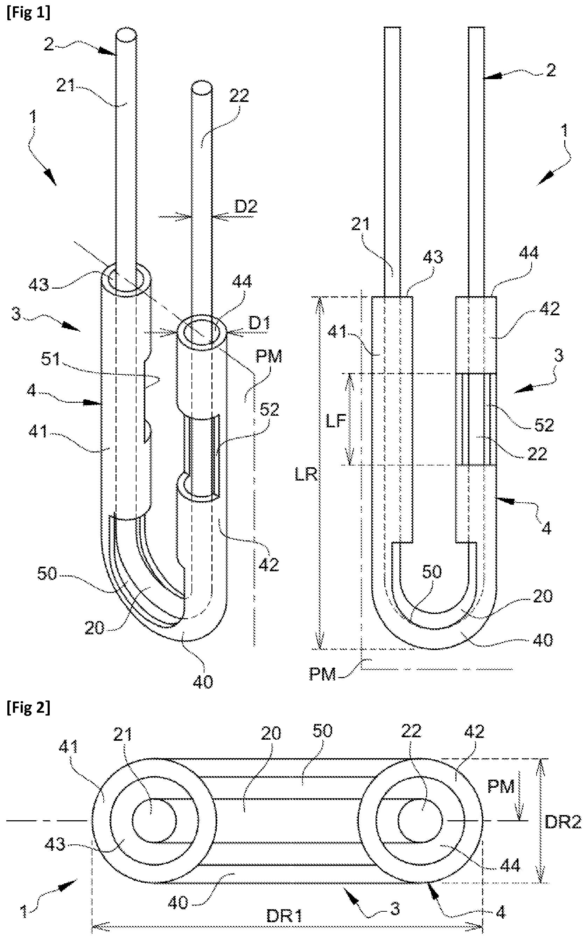

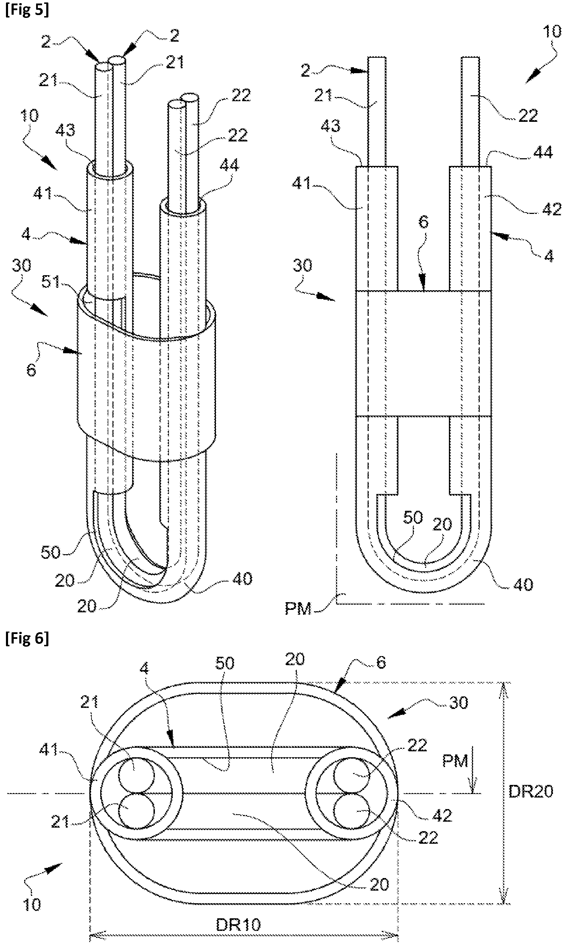

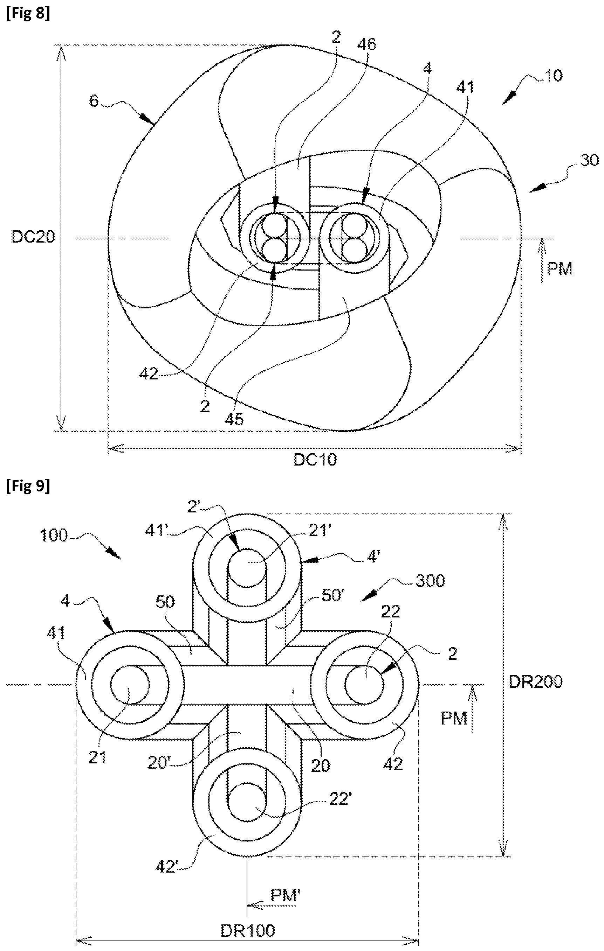

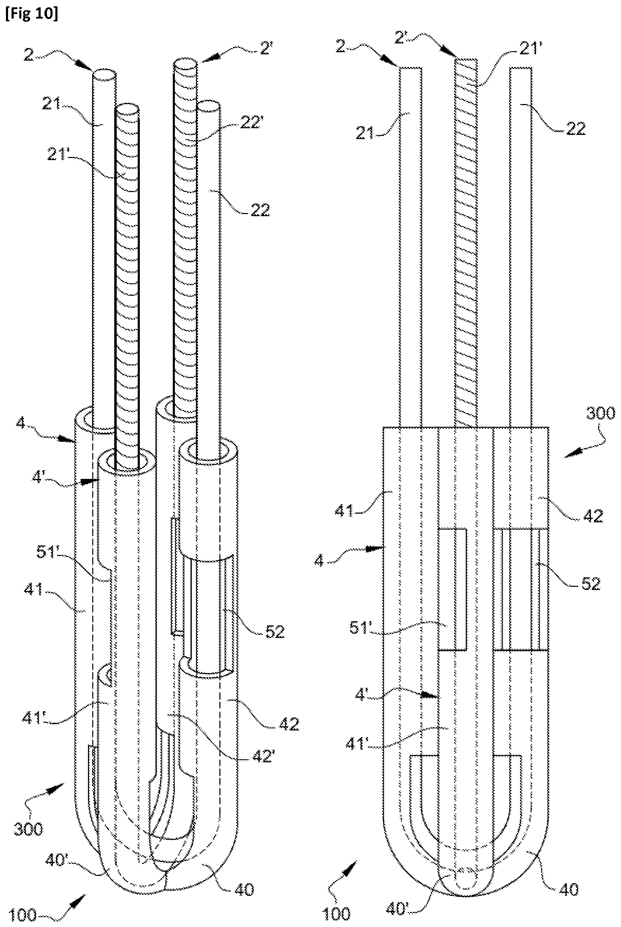

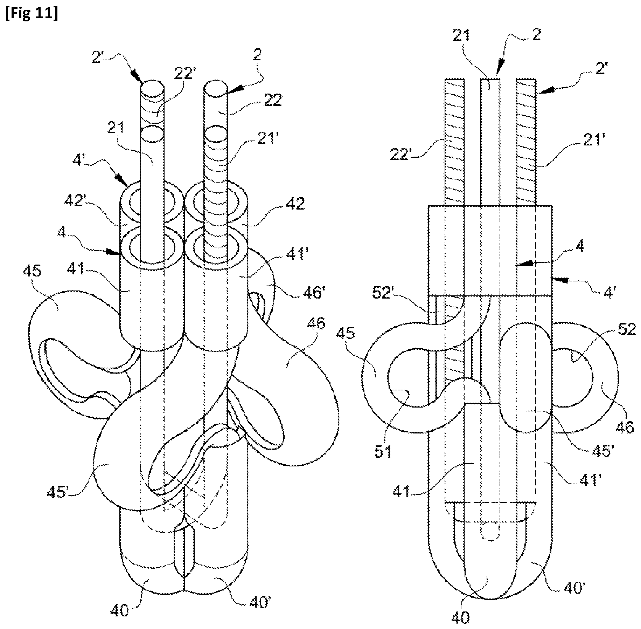

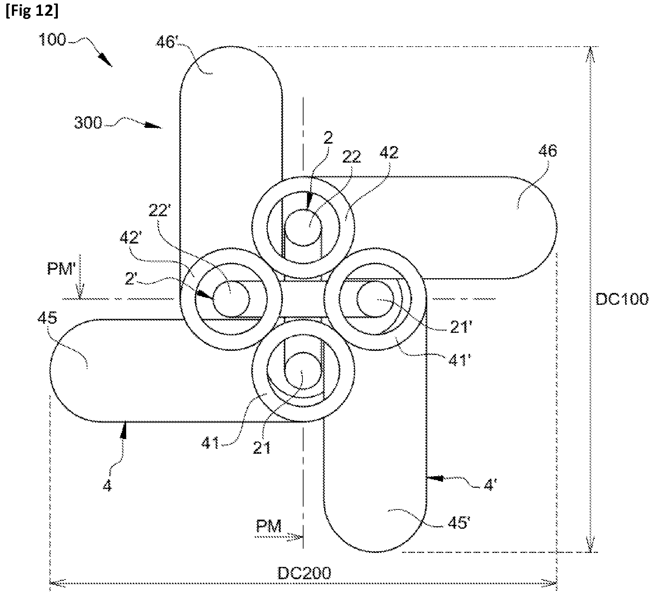

Les Figures 1 à 4 se rapportent à une première ancre de suture 1 conforme à l'invention, les Figures 5 à 8 se rapportent à une deuxième ancre de suture 10 conforme à l'invention et les Figures 9 à 12 se rapportent à une troisième ancre de suture 100 conforme à l'invention. Pour la suite de la description, les mêmes références numériques seront généralement employées pour les éléments structurels ou fonctionnels qui sont identiques ou similaires dans les trois ancres de suture 1, 10, 100.

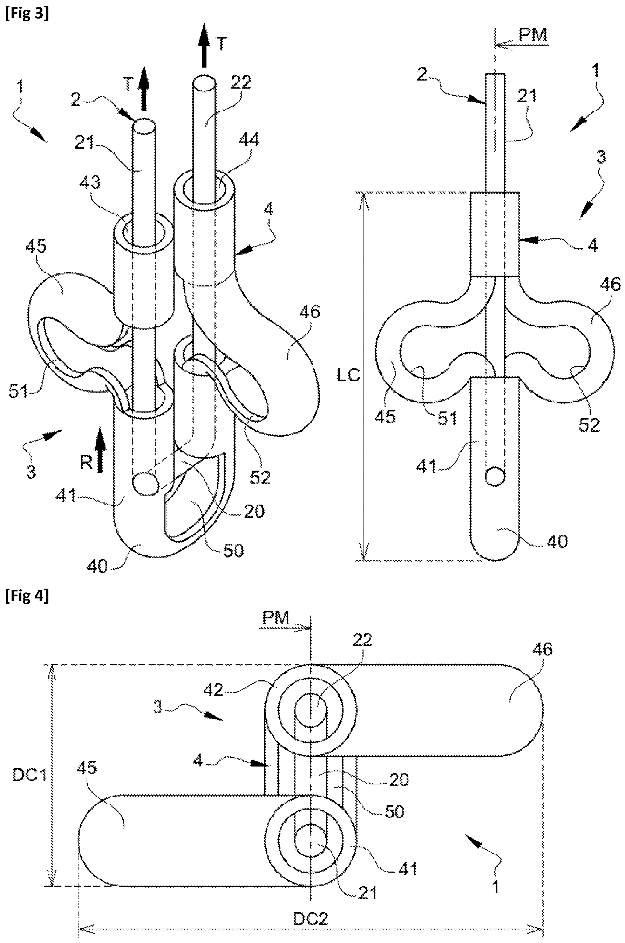

L'ancre de suture 1, 10, 100 comprend un fil de suture 2 accouplé à un élément d'ancrage compressible 3, 30, 300 et cette ancre de suture 1, 10, 100 est configurable entre :
- une configuration de repos (illustrée sur les Figures 1, 2, 5, 6, 9 et 10) dans laquelle l'élément d'ancrage compressible 3, 30, 300 est au repos pour permettre une insertion à l'intérieur d'un trou ménagé dans l'os ; et
- une configuration comprimée (illustrée sur les Figures 3, 4, 7, 8, 11 et 12) dans laquelle l'élément d'ancrage compressible 3, 30, 300 est comprimé pour permettre un blocage à l'intérieur du trou ménagé dans l'os et un ancrage du fil de suture.

Le fil de suture 2 comprend un corps qui est tissé ou tressé avec des fibres réalisées dans un ou plusieurs matériaux biocompatibles, comme par exemple un ou plusieurs matériaux biocompatibles choisis dans la liste comprenant : polyéthylène, polyester, polyhydroxyalkanoate, et une combinaison d'au moins certains d'entre eux.

L'élément d'ancrage compressible 3, 30, 300 comprend au moins une gaine compressible 4 qui se présente sous la forme d'un corps tubulaire allongé (en forme de boudin), où ce corps est tissé ou tressé avec des fibres réalisées dans un ou plusieurs matériaux biocompatibles, comme par exemple un ou plusieurs matériaux biocompatibles choisis dans la liste comprenant : polyéthylène (par exemple polyéthylène à très haut poids moléculaire dit « UHMWPE »), polyester, polyhydroxyalkanoate, et une combinaison d'au moins certains d'entre eux.

Il est envisageable que les fibres du corps de la gaine compressible 4 et les fibres du corps du fil de suture 2 soient réalisées dans au moins un même matériau biocompatible.

Comme visible sur les Figures 1, 5 et 10, en configuration de repos, la gaine compressible 4 présente une forme générale d'arche en « U » avec une section coudée centrale 40 reliant entre elles deux sections longitudinales 41, 42 espacées l'une de l'autre et se terminant par des terminaisons libres munies d'une première ouverture 43 et d'une seconde ouverture 44 respectives, avec une première section longitudinale 41 et une seconde section longitudinale 42.

Autrement dit, la gaine compressible 4 se présente sous la forme d'un boudin coudée ou repliée sur lui-même pour avoir une forme d'arche. Cette gaine compressible 4 présente un premier diamètre D1 (illustré sur la Figure 1), qui est constant sur toute sa longueur (ou au moins 90% de sa longueur), de sorte que les sections longitudinales 41, 42 présentent également ce premier diamètre D1. Ce premier diamètre D1, qui est un diamètre externe, est par exemple compris entre 0,8 et 1,3 millimètres.

Par ailleurs, le fil de suture 2 présente un second diamètre D2 (illustré sur la Figure 2) qui est inférieur au premier diamètre D1, avec un quotient du premier diamètre sur le second diamètre qui est au moins supérieur à 2, autrement dit D1/D2 est supérieur ou égal à 2. Ce second diamètre D2, qui est un diamètre externe, est par exemple compris entre 0,3 et 0,6 millimètres. Dans une réalisation particulière, le premier diamètre D1 est compris entre 1,10 et 1,20 millimètres, et le second diamètre D2 est compris entre 0,50 et 0,60 millimètres.

Comme visible sur les Figures, le fil de suture 2 traverse la gaine compressible 4 en entrant par la première ouverture 43 et en ressortant par la seconde ouverture 44, et par ailleurs ce fil de suture 2 est libre de coulisser dans la gaine compressible 4. Ainsi, ce fil de suture 2 présente un premier brin entrant à l'intérieur de la première section longitudinale 41 de la gaine compressible 4 au travers de la première ouverture 43, et un second brin 22 prolongeant le premier brin 21 et ressortant de la seconde section longitudinale 42 de la gaine compressible 4 au travers de la seconde ouverture 44. Le fil de suture 2 présente également une section coudée 20 reliant le premier brin 21 au second brin 22, où cette section coudée s'étend à l'intérieur de la section coudée centrale 40 de la gaine compressible 4.

Par ailleurs, la gaine compressible 4 présente :
- une première fenêtre 51 disposée dans la première section longitudinale 41 et ouvrant sur le premier brin 21 ; et
- une seconde fenêtre 52 disposée dans la seconde section longitudinale 42 et ouvrant sur le second brin 22.

Ces fenêtres 51, 52 sont donc des trous formés dans le corps de la gaine compressible 4, mettant ainsi partiellement à nu le premier brin 41 et le second brin 42 du fil de suture 2 à l'intérieur des sections longitudinales 41, 42.

En configuration de repos, la première fenêtre 51 et la seconde fenêtre 52 de la gaine compressible 4 sont disposées de part et d'autre d'un plan médian PM de la gaine compressible 4 dans lequel sont inclus la section coudée centrale 40, la première section longitudinale 41 et la seconde section longitudinale 42 de la gaine compressible 4. Ce plan médian PM de la gaine compressible 4 passent donc au travers de la section coudée centrale 40, de la première section longitudinale 41 et de la seconde section longitudinale 42.

Ces fenêtres 51, 52 sont situées au même niveau, autrement dit à la même distance de la section coudée centrale 40, et elles présentent sensiblement la même longueur, appelée longueur de fenêtre LF (illustrée sur la Figure 1) mesurée dans le sens de la longueur de la première section longitudinale 41 et de la seconde section longitudinale 42 respectivement. En d'autres termes, la longueur de fenêtre LF est de même valeur pour la première fenêtre 51 et la seconde fenêtre 52, à plus ou moins 10% près.

Ces fenêtres 51, 52 forment des amorces de pliage pour la première section longitudinale 41 et de la seconde section longitudinale 42, avec des pliages antagonistes du fait que les fenêtres 51, 52 soit positionnées de part et d'autre du plan médian PM de la gaine compressible 4.

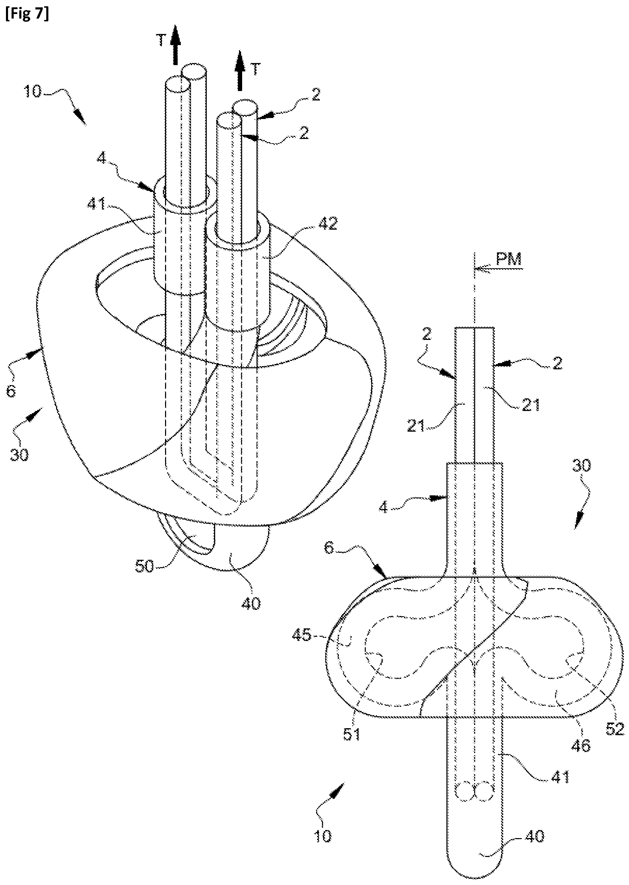

L'ancre de suture 1, 10, 100 est inséré dans l'os, plus spécifiquement à l'intérieur du trou ménagé dans l'os, en étant en configuration de repos. Ensuite, sous l'effet d'une tension exercée par le fil de suture 2 en tirant sur les deux brins 21, 22 (comme schématisé par les flèches T sur les Figures 3 et 7), ce fil de suture 2 va agir sur les deux sections longitudinales 41, 42 de la gaine compressible 4 pour passer en configuration comprimée.

En effet, en tirant sur les deux brins 21, 22, la section coudée centrale 40 remonte (comme schématisé par la flèche R sur la Figure 3), tandis que les terminaisons libres des sections longitudinales 41, 42 ne bougent pas ou peu (en étant coincé par exemple par un outil adéquat qui bloque la gaine compressible 4 en l'empêchant de ressortir du trou), et ainsi les sections longitudinales 41, 42 se compriment (et raccourcissent) et, en ce comprimant, elles se déforment au niveau des fenêtres 51, 52 pour former des plis saillants 45, 46.

Comme visible sur les Figures 3, 7 et 11, en configuration comprimée, la première section longitudinale 41 et la seconde section longitudinale 42 de la gaine compressible 4 sont comprimées en présentant un premier pli saillant 45 et un second pli saillant 46 formés respectivement au niveau de la première fenêtre 51 et de la seconde fenêtre 52. Ces plis saillants 45, 46 saillent hors du plan médian PM, dans des directions opposées, et forment ainsi des zones d'appui antagonistes pour l'élément d'ancrage compressible 3, 30, 300, ou la gaine compressible 4.

Ces plis saillants 45, 46 vont contribuer à ce que les deux sections longitudinales 41, 42 s'enroulement sur elles-mêmes durant la compression concomitamment à la compression axiale exercée en tirant sur les brins 21, 22 du fil de suture 2 de sorte que, dans la configuration comprimée, les deux sections longitudinales 41, 42 sont comprimées l'une contre l'autre selon une conformation en hélice (non illustrée sur les Figures), tandis que les brins 21, 22 du fil de suture 2 seront substantiellement rectilignes et parallèles.

De manière optionnelle, la gaine compressible 4 présente une fenêtre centrale 50 disposée dans la section coudée centrale 40, sur une portion intérieure de coude, ouvrant sur la section coudée 20 du fil de suture 2. Cette fenêtre centrale 50 est donc prévue à l'intérieur du coude (ouverture tournée vers les terminaisons libres des sections longitudinales) et met à nu partiellement la section coudée 20. Ainsi, et comme visible sur la Figure 3, lorsqu'on tire sur les deux brins 21, 22, la section coudée 20 du fil de suture 2 passe à travers cette fenêtre centrale 50, et remonte jusqu'aux bords de la fenêtre centrale 50 avant de comprimer les deux sections longitudinales 41, 42 et ainsi les faire plier au niveau des fenêtres 51, 52.

En configuration de repos, l'élément d'ancrage compressible 3, 30, 300, ou la gaine compressible 4, présente une première valeur de dimension longitudinale LR mesurée longitudinalement depuis la section coudée centrale 40 jusqu'à la terminaison libre de la première section longitudinale 41 ou de la seconde section longitudinale 42. Dans les exemples illustrés, le ratio entre la longueur de fenêtre LF et la première valeur de dimension longitudinale LR, soit LF/LR, est compris entre 0,1 et 0,4. Par contre, en configuration comprimée, l'élément d'ancrage compressible 3, 30, 300, ou la gaine compressible 4, présente une seconde valeur de dimension longitudinale inférieure à la première valeur de dimension longitudinale LR, de sorte que l'élément d'ancrage compressible 3, 30, 300 est bien comprimé axialement en étant plus court.

En configuration de repos, l'élément d'ancrage compressible 3, 30, 300, ou la gaine compressible 4, présente une première valeur de dimension transversale DR1, DR10, DR100 (illustrée sur les Figures 2, 6 et 9) mesurée dans le plan médian PM et une autre première valeur de dimension transversale DR2, DR20, DR200 (illustrée sur les Figures 2, 6 et 9) mesurée orthogonalement au plan médian PM. Par contre, dans la configuration comprimée, l'élément d'ancrage compressible 3, 30, 300, ou la gaine compressible 4, présente :
- une seconde valeur de dimension transversale DC1, DC10, DC100 (illustrée sur les Figures 4, 8 et 12) mesurée dans le plan médian PM et qui est supérieure ou égale à la première valeur de dimension transversale DR1, DR10, DR100 (dans les trois ancres 1, 10, 100 on observe DC1=DR1, DC10>DR10 et DC100>DR100) ;
- une autre seconde valeur de dimension transversale DC2, DC20, DC200 (illustrée sur les Figures 4, 8 et 12) mesurée orthogonalement au plan médian PM et qui est supérieure à l'autre première valeur de dimension transversale DR2, DR20, DR200 (dans les trois ancres 1, 10, 100 on observe DC2>DR2, DC20>DR20 et DC200>DR200).

De la sorte, l'élément d'ancrage compressible 3, 30, 300 se comprime et s'élargit en même temps pour se coincer à l'intérieur du trou ménagé dans l'os.

Dans le premier mode de réalisation des Figures 1 à 4, l'élément d'ancrage compressible 3 de la première ancre de suture 1 comprend une seule gaine compressible 4. Ainsi, cet élément d'ancrage compressible 3 est formé de cette seulegaine compressible 4 traversée par un fil de suture 2, voire deux fils de suture.

Dans le deuxième mode de réalisation des Figures 5 à 8, l'élément d'ancrage compressible 30 de la deuxième ancre de suture 10 comprend une seule gaine compressible 4 solidaire d'un anneau extensible 6. Ainsi, cet élément d'ancrage compressible 30 est formé de l'ensemble comprenant la gaine compressible 4 et l'anneau extensible 6 ; cette graine compressible 4 étant traversée par un fil de suture 2, voire deux fils de suture 2. Dans l'exemple illustré, la graine compressible 4 est traversée par deux fils de suture 2. L'anneau extensible 6 est disposé autour de la gaine compressible 4, au même niveau que les fenêtres 51, 52, de sorte que cet anneau extensible 6 vient en regard de la première fenêtre 51 et de la seconde fenêtre 52.

Cet anneau extensible 6 peut être fixé à l'arrière des fenêtres 51, 52, sur les parties qui formeront les plis saillants 45, 46 en configuration comprimée. Dans la configuration comprimée, les plis saillants 45, 46 poussent sur l'anneau extensible 6 qui va se dilater ou se déformer, comme visible sur les Figures 7 et 8.

L'anneau extensible 6 peut être formé d'un corps tissé ou tressé avec des fibres réalisées dans un ou plusieurs matériaux biocompatibles, comme par exemple un ou plusieurs matériaux biocompatibles choisis dans la liste comprenant : polyéthylène (par exemple polyéthylène à très haut poids moléculaire dit « UHMWPE »), polyester, polyhydroxyalkanoate, et une combinaison d'au moins certains d'entre eux. Il est envisageable que les fibres du corps de la gaine compressible 4 et les fibres du corps de l'anneau extensible 6 soient réalisées dans au moins un même matériau biocompatible.

Dans le troisième mode de réalisation des Figures 9 à 12, l'élément d'ancrage compressible 300 de la troisième ancre de suture 10 comprend deux gaines compressibles 4 solidaires l'une de l'autre au niveau de leurs sections centrales coudées 40, 40'. Chaque gaine compressible 4, 4' reçoit intérieurement au moins un fil de suture 2, 2' respectif.

L'autre gaine compressible 4' est identique à la gaine compressible 4 déjà décrite, en présentant deux sections longitudinales 41', 42' recevant les brins 21', 22' du fil de suture 2' et munies de fenêtres 51', 52' respectives, où ces sections longitudinales 41', 42' sont reliées par une section centrale coudée 40' recevant la section coudée 20' du fil de suture 2' et munie d'une fenêtre centrale. Ainsi, en configuration comprimée, cette autre gaine compressible 4' présente des plis saillants 45', 46' au niveau des fenêtres 51', 52'. Comme visible sur la Figure 9, les plans médians PM, PM' des deux gaines compressibles 4, 4' sont orthogonaux de sorte que, en configuration comprimée, l'élément d'ancrage compressible 300 présente une forme générale en croix, chaque pli saillant 45, 46, 45', 46' formant une branche de la croix.

L'ancre ancre de suture 1, 10, 100 peut être incluse dans un kit de suture comprenant une ou plusieurs ancres de suture 1, 10, 100 et comprenant des ancillaires ou outils chirurgicaux qui serviront à guider et à insérer l'ancre de suture 1, 10, 100 lorsqu'en configuration de repos à l'intérieur du trou ménagé dans l'os, ces ancillaires ou outils chirurgicaux comprenant au moins :
- un instrument d'insertion propre à insérer l'ancre de suture 1, 10, 100 dans le trou de l'os, où cet instrument d'insertion comprend une partie distale propre à passer entre les deux brins 21, 22 et également entre les deux sections longitudinales 41, 42 de la gaine compressible 4, et se terminant par une extrémité libre propre à venir en contact avec la section coudée centrale 40 de la gaine compressible 4 (en configuration de repos) afin de prendre appui sur celle-ci et permettre d'exercer un effort de poussée (vers le bas) ; et
- un tube de guidage propre à venir en appui sur les terminaisons libres des sections longitudinales 41, 42 de la gaine compressible 4 (bloquant ainsi celle-ci pour l'empêcher de remonter), et présentant un canal intérieur longitudinal dimensionné pour un passage à la fois des deux brins 21, 22 du fil de suture 2 (ou des fils de suture 2) ainsi que de la partie distale de l'instrument d'insertion.

L'extrémité libre de l'instrument d'insertion se présente avantageusement sous la forme d'une fourche à deux bras , avec la section coudée centrale 40 de la gaine compressible 4 qui se loge dans le creux de la fourche entre les deux bras. Ces deux bras peuvent se terminer par des pointes qui dépassent de la section coudée centrale 40 de la gaine compressible 4. Ainsi, les pointes des bras permettent de former à l'extrémité libre de l'instrument d'insertion une extrémité auto-perforante, ce qui permet de ménager le trou par impaction en même temps que l'ancre de suture 1, 10, 100 est inséré dans le trou.

Le tube de guidage permet également de pousser sur l'élément d'ancrage compressible 3, 30, 300 lors de son insertion dans le trou, tout en guidant l'instrument d'insertion, ce qui particulièrement pratique si l'instrument d'insertion est inséré par impaction. Les brins 21, 22 du ou des fils de suture 2 dépassent bien entendu du tube de guidage pour permettre leur manipulation. Une fois que l'ancre de suture 1, 10, 100 est insérée intégralement dans le trou, l'instrument d'insertion est remonté au moins partiellement pour le dégager complètement de l'ancre de suture 1, 10, 100, et le chirurgien peut alors tirer sur les brins 21, 22 (comme schématisé par les flèches T sur les Figures 3 et 7), alors que le tube de guidage reste en place pour bloquer l'élément d'ancrage compressible 3, 30, 300 ce qui va amener l'ancre de suture 1, 10, 100 en configuration comprimée à l'intérieur du trou ménagé dans l'os.

A l'issue de cette étape, le tube de guidage et l'instrument d'insertion peuvent être retirés, laissant l'ancre de suture 1, 10, 100 bloquée dans le trou en configuration comprimée, et avec les brins 21, 22 du fil de suture 2 qui sont libres de coulisser dans les deux sections longitudinales 41, 42 de la gaine compressible 4 en configuration comprimée.

## Revendications

1. Ancre de suture (1 ; 10 ; 100), pour un ancrage d'un tissu mou sur un os, ladite ancre de suture (1 ; 10 ; 100) comprenant au moins un fil de suture (2) accouplé à un élément d'ancrage compressible (3 ; 30 ; 300), ledit élément d'ancrage compressible (3 ; 30 ; 300) comprenant au moins une gaine compressible (4) traversée par ledit fil de suture (2), ladite ancre de suture (1 ; 10 ; 100) étant configurable entre :
- une configuration de repos dans laquelle l'élément d'ancrage compressible (3 ; 30 ; 300) est au repos ; et
- une configuration comprimée dans laquelle l'élément d'ancrage compressible (3) est comprimé,
dans laquelle, en configuration de repos :
- la gaine compressible (4) présente une forme générale d'arche en « U » avec une section coudée centrale (40) reliant entre elles une première section longitudinale (41) et une seconde section longitudinale (42) espacées l'une de l'autre et se terminant par des terminaisons libres munies d'une première ouverture (43) et d'une seconde ouverture (44) respectives ; et
- le fil de suture (2) est libre de coulisser à l'intérieur de la gaine compressible (4) et présente un premier brin (21) entrant à l'intérieur de la première section longitudinale (41) de la gaine compressible (4) au travers de la première ouverture (43), et un second brin (22) prolongeant le premier brin (21) et ressortant de la seconde section longitudinale (42) de la gaine compressible (4) au travers de la seconde ouverture (44) ;
et dans laquelle la gaine compressible (4) présente :
- une première fenêtre (51) disposée dans la première section longitudinale (41) et ouvrant sur le premier brin (21) ; et
- une seconde fenêtre (52) disposée dans la seconde section longitudinale (42) et ouvrant sur le second brin (22),
**caractérisé par**, en configuration de repos, la première fenêtre (51) et la seconde fenêtre (52) de la gaine compressible (4) sont disposées de part et d'autre d'un plan médian (PM) de la gaine compressible (4) dans lequel sont inclus la section coudée centrale (40), la première section longitudinale (41) et la seconde section longitudinale (42) de ladite gaine compressible (4).

2. Ancre de suture (1 ; 10 ; 100) selon la revendication 1, dans laquelle la gaine compressible (4) présente une fenêtre centrale (50) disposée dans la section coudée centrale (40), sur une portion intérieure de coude, ouvrant sur une section coudée (20) du fil de suture (2) reliant le premier brin (21) au second brin (22).

3. Ancre de suture (1; 10 ; 100) selon les revendications 1 ou 2, dans laquelle, en configuration de repos, la première fenêtre (51) et la seconde fenêtre (52) s'étendent chacune sur une longueur de fenêtre (LF) mesurée dans le sens de la longueur de la première section longitudinale (41) et de la seconde section longitudinale (42) respectives, où cette longueur de fenêtre (LF) est de même valeur pour la première fenêtre (51) et la seconde fenêtre (52), à plus ou moins 10% près.

4. Ancre de suture (1; 10 ; 100) selon la revendication 3, dans laquelle, en configuration de repos, la gaine compressible (4) présente une première valeur de dimension longitudinale (LR) mesurée longitudinalement depuis la section coudée centrale (40) jusqu'à la terminaison libre de la première section longitudinale (41) ou de la seconde section longitudinale (42), et le ratio entre la longueur de fenêtre (LF) et la première valeur de dimension longitudinale (LR) est compris entre 0,1 et 0,4.

5. Ancre de suture (1 ; 10 ; 100) selon l'une quelconque des revendications 1 à 4, dans laquelle la gaine compressible (4) reçoit intérieurement un ou deux fils de suture (2).

6. Ancre de suture (1; 10) selon l'une quelconque des revendications 1 à 5, dans laquelle l'élément d'ancrage compressible (3 ; 30) comprend une seule gaine compressible (4).

7. Ancre de suture (10) selon la revendication 6, dans laquelle l'élément d'ancrage compressible (30) comprend un anneau extensible (6) solidaire de la gaine compressible (4), ledit anneau extensible (6) étant disposé autour de la gaine compressible (4) et venant en regard de la première fenêtre (51) et de la seconde fenêtre (52).

8. Ancre de suture (100) selon l'une quelconque des revendications 1 à 5, dans laquelle l'élément d'ancrage compressible (300) comprend deux gaines compressibles (4, 4') solidaires l'une de l'autre au niveau de leurs sections coudées centrales (40, 40') respectives et dont les plans médians (PM, PM') sont orthogonaux, chaque gaine compressible (4, 4') recevant intérieurement au moins un fil de suture (2, 2').

9. Ancre de suture (1 ; 10 ; 100) selon l'une quelconque des revendications précédentes, dans laquelle les sections longitudinales (41, 42) de la gaine compressible (4) présentent un premier diamètre et le fil de suture (2) présente un second diamètre (D2) qui est inférieur au premier diamètre (D1), avec un quotient du premier diamètre (D1) sur le second diamètre (D2) qui est au moins supérieur à 2.

10. Ancre de suture (1; 10 ; 100) selon l'une quelconque des revendications précédentes, dans laquelle, en configuration comprimée, la première section longitudinale (41) et la seconde section longitudinale (42) de la gaine compressible (4) sont comprimées en présentant un premier pli saillant (45) et un second pli saillant (46) formés respectivement au niveau de la première fenêtre (51) et de la seconde fenêtre (52).

11. Ancre de suture (1 ; 10; 100) selon l'une quelconque des revendications précédentes, dans laquelle, en configuration comprimée, la première section longitudinale (41) et la seconde section longitudinale (42) de la gaine compressible (4) sont comprimées l'une contre l'autre selon une conformation en hélice.

12. Kit de suture, pour un ancrage d'un tissu mou sur un os, ledit kit de suture comprenant au moins une ancre de suture (1; 10 ; 100) selon l'une quelconque des revendications précédentes, et au moins un instrument d'insertion propre à insérer ladite ancre de suture (1 ; 10 ; 100) lorsqu'en configuration de repos à l'intérieur d'un trou ménagé dans l'os.

13. Kit de suture selon la revendication 12, dans lequel l'instrument d'insertion comprend une partie distale propre à passer entre la première section longitudinale (41) et la seconde section longitudinale (42) de la gaine compressible (4), et se terminant par une extrémité libre propre à venir en contact avec la section coudée centrale (40) de la gaine compressible (4) afin de prendre appui sur celle-ci et permettre d'exercer un effort de poussée.

14. Kit de suture selon la revendication 13, dans lequel l'extrémité libre de l'instrument d'insertion se présente sous la forme d'une fourche à deux bras.

15. Kit de suture selon l'une quelconque des revendications 12 à 14, comprenant en outre un tube de guidage propre à venir en appui sur les terminaisons libres de la première section longitudinale (41) et de la seconde section longitudinale (42) de la gaine compressible (4), et présentant un canal intérieur longitudinal dimensionné pour un passage du premier brin (21) et du second brin (22) du fil de suture (2) et de l'instrument d'insertion.

## Patentansprüche

1. Nahtanker (1; 10; 100) zur Verankerung von Weichgewebe an einem Knochen, wobei der Nahtanker (1; 10; 100) mindestens einen Nahtfaden (2) umfasst, der mit einem komprimierbaren Verankerungselement (3; 30; 300) gekoppelt ist, wobei das komprimierbare Verankerungselement (3; 30; 300) mindestens eine komprimierbare Hülle (4) umfasst, durch die der Nahtfaden (2) hindurch verläuft, wobei der Nahtanker (1; 10; 100) zwischen Folgendem konfigurierbar ist:
- einer Ruhekonfiguration, in der das komprimierbare Verankerungselement (3; 30; 300) ruht; und
- einer komprimierten Konfiguration, in der das komprimierbare Verankerungselement (3) komprimiert wird,
wobei in der Ruhekonfiguration:
- die komprimierbare Hülle (4) eine allgemeine "U"-förmige Bogenform mit einem mittleren gekrümmten Abschnitt (40) aufweist, der einen ersten Längsabschnitt (41) und einen zweiten Längsabschnitt (42) miteinander verbindet, die voneinander beabstandet sind und mit freien Abschlüssen mit einer jeweiligen ersten Öffnung (43) und einer jeweiligen zweiten Öffnung (44) abschließen; und
- der Nahtfaden (2) frei in dem Inneren der komprimierbaren Hülle (4) gleiten kann und einen ersten Strang (21), der durch die erste Öffnung (43) in den ersten Längsabschnitt (41) der komprimierbaren Hülle (4) eindringt, und einen zweiten Strang (22) aufweist, der den ersten Strang (21) verlängert und durch die zweite Öffnung (44) aus dem zweiten Längsabschnitt (42) der komprimierbaren Hülle (4) herausragt;
und wobei die komprimierbare Hülle (4) Folgendes aufweist:
- ein erstes Fenster (51), das in dem ersten Längsabschnitt (41) angeordnet ist und sich zu dem ersten Strang (21) hin öffnet; und
- ein zweites Fenster (52), das in dem zweiten Längsabschnitt (42) angeordnet ist und sich zu dem zweiten Strang (22) hin öffnet,
**gekennzeichnet dadurch, dass**
in der Ruhekonfiguration das erste Fenster (51) und das zweite Fenster (52) der komprimierbaren Hülle (4) auf beiden Seiten einer Mittelebene (PM) der komprimierbaren Hülle (4) angeordnet sind, in der der mittlere gekrümmte Abschnitt (40), der erste Längsabschnitt (41) und der zweite Längsabschnitt (42) der komprimierbaren Hülle (4) enthalten sind.

2. Nahtanker (1; 10; 100) nach Anspruch 1, wobei die komprimierbare Hülle (4) ein zentrales Fenster (50) aufweist, das in dem mittleren gekrümmten Abschnitt (40) an einem inneren gekrümmten Abschnitt angeordnet ist und sich zu einem gekrümmten Abschnitt (20) des Nahtfadens (2) öffnet, der den ersten Strang (21) mit dem zweiten Strang (22) verbindet.

3. Nahtanker (1; 10; 100) nach den Ansprüchen 1 oder 2, wobei sich in der Ruhekonfiguration das erste Fenster (51) und das zweite Fenster (52) jeweils über eine in der Richtung der Länge des ersten Längsabschnitts (41) bzw. des zweiten Längsabschnitts (42) gemessene Fensterlänge (LF) erstrecken, wobei diese Fensterlänge (LF) für das erste Fenster (51) und das zweite Fenster (52) etwa 10 % genau gleich ist.

4. Nahtanker (1; 10; 100) nach Anspruch 3, wobei die komprimierbare Hülle (4) in der Ruhekonfiguration einen ersten Längsmaßwert (LR) aufweist, der längs von dem mittleren gekrümmten Abschnitt (40) bis zu dem freien Abschluss des ersten Längsabschnitts (41) oder des zweiten Längsabschnitts (42) gemessen wird, und das Verhältnis zwischen der Fensterlänge (LF) und dem ersten Längsmaßwert (LR) zwischen 0,1 und 0,4 liegt.

5. Nahtanker (1; 10; 100) nach einem der Ansprüche 1 bis 4, wobei die komprimierbare Hülle (4) innen ein oder zwei Nahtfäden (2) aufnimmt.

6. Nahtanker (1; 10) nach einem der Ansprüche 1 bis 5, wobei das komprimierbare Verankerungselement (3; 30) eine einzige komprimierbare Hülle (4) umfasst.

7. Nahtanker (10) nach Anspruch 6, wobei das komprimierbare Verankerungselement (30) einen dehnbaren Ring (6) umfasst, der mit der komprimierbaren Hülle (4) fest verbunden ist, wobei der dehnbare Ring (6) um die komprimierbare Hülle (4) herum angeordnet ist und gegenüber dem ersten Fenster (51) und dem zweiten Fenster (52) steht.

8. Nahtanker (100) nach einem der Ansprüche 1 bis 5, wobei das komprimierbare Verankerungselement (300) zwei komprimierbare Hüllen (4, 4') umfasst, die an ihren jeweiligen mittleren gekrümmten Abschnitten (40, 40') fest miteinander verbunden sind und deren Mittelebenen (PM, PM') orthogonal sind, wobei jede komprimierbare Hülle (4, 4') innen mindestens einen Nahtfaden (2, 2') aufnimmt.

9. Nahtanker (1; 10; 100) nach einem der vorhergehenden Ansprüche, wobei die Längsabschnitte (41, 42) der komprimierbaren Hülle (4) einen ersten Durchmesser aufweisen und der Nahtfaden (2) einen zweiten Durchmesser (D2) aufweist, der kleiner als der erste Durchmesser (D1) ist, mit einem Quotienten aus dem ersten Durchmesser (D1) und dem zweiten Durchmesser (D2), der mindestens größer als 2 ist.

10. Nahtanker (1; 10; 100) nach einem der vorhergehenden Ansprüche, wobei der erste Längsabschnitt (41) und der zweite Längsabschnitt (42) der komprimierbaren Hülle (4) in der komprimierten Konfiguration komprimiert werden und eine erste vorstehende Falte (45) und eine zweite vorstehende Falte (46) aufweisen, die jeweils auf Höhe des ersten Fensters (51) und des zweiten Fensters (52) ausgebildet sind.

11. Nahtanker (1; 10; 100) nach einem der vorhergehenden Ansprüche, wobei der erste Längsabschnitt (41) und der zweite Längsabschnitt (42) der komprimierbaren Hülle (4) in der komprimierten Konfiguration in einer Helixform gegeneinander komprimiert werden.

12. Nahtset zur Verankerung von Weichgewebe an einem Knochen, wobei das Nahtset mindestens einen Nahtanker (1; 10; 100) nach einem der vorhergehenden Ansprüche und mindestens ein Einsetzinstrument umfasst, das geeignet ist, den Nahtanker (1; 10; 100), wenn er sich in der Ruhekonfiguration befindet, in ein Loch in dem Knochen einzuführen.

13. Nahtset nach Anspruch 12, wobei das Einsetzinstrument einen distalen Teil umfasst, der geeignet ist, zwischen dem ersten Längsabschnitt (41) und dem zweiten Längsabschnitt (42) der komprimierbaren Hülle (4) zu verlaufen, und in einem freien Ende endet, das geeignet ist, mit dem mittleren gekrümmten Abschnitt (40) der komprimierbaren Hülle (4) in Kontakt zu kommen, um sich auf diesem abzustützen und das Ausüben einer Schubkraft zu ermöglichen.

14. Nahtset nach Anspruch 13, wobei das freie Ende des Einsetzinstruments in Form einer zweiarmigen Gabel ausgebildet ist.

15. Nahtset nach einem der Ansprüche 12 bis 14, ferner umfassend ein Führungsrohr, das geeignet ist, an den freien Abschlüssen des ersten Längsabschnitts (41) und des zweiten Längsabschnitts (42) der komprimierbaren Hülle (4) aufzuliegen, und einen inneren Längskanal aufweist, der für den Durchgang des ersten Strangs (21) und des zweiten Strangs (22) des Nahtfadens (2) und des Einsetzinstruments dimensioniert ist.

## Claims

1. A suture anchor (1; 10; 100), for anchoring a soft tissue to a bone, said suture anchor (1; 10; 100) comprising at least one suture (2) coupled to a compressible anchoring element (3; 30; 300), said compressible anchoring element (3; 30; 300) comprising at least one compressible sheath (4) through which said suture (2) passes, said suture anchor (1; 10; 100) being configurable between:
- a rest configuration in which the compressible anchoring element (3; 30; 300) is at rest; and
- a compressed configuration in which the compressible anchoring element (3) is compressed,
wherein, in the rest configuration:
- the compressible sheath (4) has a general U-shaped arch form with a central bent section (40) connecting a first longitudinal section (41) and a second longitudinal section (42) spaced apart from each other and ending in free terminations provided with respective first opening (43) and second opening (44); and
- the suture (2) is free to slide inside the compressible sheath (4) and has a first strand (21) entering the first longitudinal section (41) of the compressible sheath (4) through the first opening (43), and a second strand (22) extending from the first strand (21) and exiting the second longitudinal section (42) of the compressible sheath (4) through the second opening (44);
and wherein the compressible sheath (4) has:
- a first window (51) arranged in the first longitudinal section (41) and opening onto the first strand (21); and
- a second window (52) arranged in the second longitudinal section (42) and opening onto the second strand (22),
**characterized by**,
in the rest configuration, the first window (51) and the second window (52) of the compressible sheath (4) are arranged on either side of a median plane (PM) of the compressible sheath (4) in which are included the central bent section (40), the first longitudinal section (41) and the second longitudinal section (42) of said compressible sheath (4).

2. The suture anchor (1; 10; 100) according to claim 1, wherein the compressible sheath (4) has a central window (50) arranged in the central bent section (40), on an inner bend portion, opening onto a bent section (20) of the suture (2) connecting the first strand (21) to the second strand (22).

3. The suture anchor (1; 10; 100) according to claim 1 or 2, wherein, in the rest configuration, the first window (51) and the second window (52) each extend over a window length (LF) measured in the lengthwise direction of the respective first longitudinal section (41) and second longitudinal section (42), where this window length (LF) has the same value for the first window (51) and the second window (52), to within plus or minus 10%.

4. The suture anchor (1; 10; 100) according to claim 3, wherein, in the rest configuration, the compressible sheath (4) has a first longitudinal dimension value (LR) measured longitudinally from the central bent section (40) to the free termination of the first longitudinal section (41) or the second longitudinal section (42), and the ratio between the window length (LF) and the first longitudinal dimension value (LR) is between 0.1 and 0.4.

5. The suture anchor (1; 10; 100) according to any one of claims 1 to 4, wherein the compressible sheath (4) internally receives one or two sutures (2).

6. The suture anchor (1; 10) according to any one of claims 1 to 5, wherein the compressible anchoring element (3; 30) comprises a single compressible sheath (4).

7. The suture anchor (10) according to claim 6, wherein the compressible anchoring element (30) comprises an expandable ring (6) secured to the compressible sheath (4), said expandable ring (6) being arranged around the compressible sheath (4) and facing the first window (51) and the second window (52).

8. The suture anchor (100) according to any one of claims 1 to 5, wherein the compressible anchoring element (300) comprises two compressible sheaths (4, 4') secured to each other at their respective central bent sections (40, 40') and whose median planes (PM, PM') are orthogonal, each compressible sheath (4, 4') internally receiving at least one suture (2, 2').

9. The suture anchor (1; 10; 100) according to any one of the preceding claims, wherein the longitudinal sections (41, 42) of the compressible sheath (4) have a first diameter and the suture (2) has a second diameter (D2) that is smaller than the first diameter (D1), with a quotient of the first diameter (D1) over the second diameter (D2) that is at least greater than 2.

10. The suture anchor (1; 10; 100) according to any one of the preceding claims, wherein, in the compressed configuration, the first longitudinal section (41) and the second longitudinal section (42) of the compressible sheath (4) are compressed, presenting a first protruding fold (45) and a second protruding fold (46) formed respectively at the first window (51) and the second window (52).

11. The suture anchor (1; 10; 100) according to any one of the preceding claims, wherein, in the compressed configuration, the first longitudinal section (41) and the second longitudinal section (42) of the compressible sheath (4) are compressed against each other in a helix conformation.

12. A suture kit, for anchoring a soft tissue to a bone, said suture kit comprising at least one suture anchor (1; 10; 100) according to any one of the preceding claims, and at least one insertion instrument adapted to insert said suture anchor (1; 10; 100) when in its rest configuration inside a hole made in the bone.

13. The suture kit according to claim 12, wherein the insertion instrument comprises a distal part adapted to pass between the first longitudinal section (41) and the second longitudinal section (42) of the compressible sheath (4), and ending in a free end adapted to come into contact with the central bent section (40) of the compressible sheath (4) in order to bear against it and allow a pushing force to be applied.

14. The suture kit according to claim 13, wherein the free end of the insertion instrument is in the form of a two-armed fork.

15. The suture kit according to any one of claims 12 to 14, further comprising a guide tube adapted to bear against the free terminations of the first longitudinal section (41) and the second longitudinal section (42) of the compressible sheath (4), and having an inner longitudinal channel sized for passage of the first strand (21) and the second strand (22) of the suture (2) and the insertion instrument.
